# EUROPEAN PATENT SPECIFICATION

(11) **EP 1 545 367 B1**
(45) Date of publication and mention of the grant of the patent: **26.12.2012**
(21) Application number: 03753681.0
(22) Date of filing: 10.09.2003
(51) Int. Cl.: F24F 5/00, A61B 19/02

(54) **STERILE AIR TROLLEY**
WAGEN MIT STERILER LUFT
CHARIOT A AIR STERILE

(30) Priority: 02.10.2002 GB 0222808
(43) Date of publication of application: 29.06.2005
(73) Proprietor: BES Rehab Limited, Fulbourn Cambridgeshire CB1 5HB (GB)
(72) Inventor: BURFOOT, Dean, Flitwick, Bedfordshire MK45 1AS (GB)
(74) Representative: Maughan, Sophie Louise
(86) International application number: PCT/GB2003/003915
(87) International publication number: WO 2004/030562

(56) References cited:
- DE-A- 2 439 778
- DE-A- 2 646 710
- DE-A- 2 916 612
- DE-A- 3 618 429
- DE-A1- 2 836 708
- GB-A- 1 481 744
- US-A- 3 629 999
- US-A- 3 721 067
- US-A- 3 881 477
- US-A- 4 531 956
- US-A- 4 996 910
- US-A- 5 817 002

## Description

### Field of the Invention

The present invention primarily concerns improvements in and relating to sterile air trolleys such as may be required for a range of different uses that require an open-topped sterile work surface, including the holding of pharmaceutical or electronic components or surgical instruments for use during a surgical operation.

### Background to the invention

Sterile air systems are used in a wide range of different applications and correspondingly take a wide range of different,forms. As a generality, however, the majority of sterile air systems involve drawing air into a unit through suction by a fan, and then direct the air through a filter, suitably of the type known as a HEPA (high efficiency particulate air) filter, and then ducting the filtered air to the required target zone whether it be a work surface or an entire room.

One particular situation in which a supply of sterile air is very important is in the surgical operating theatre of a hospital. When the hospital has extensive financial resources they will seek to condition and sterilise the air supply blown down onto the operating table. However, this is not always financially viable and does not necessarily ensure a sterile air environment in all areas of the theatre. One particular key area in the operating theatre is the area surrounding the surgical tools to be used in the operation. These surgical tools are normally presented to the surgeon on the work surface of an open topped trolley or on a tray having recently been sterilised in an autoclave. Access to these surgical tools must, of course, be unhindered during the operation. However, attempts have been made in the prior art to provide a localised sterile air environment for the surgical instruments. Prior US patents nos. US 4,531,956 and US 4,996,910 detail designs of sterile air trolley for just this purpose and which may also be used for other applications such as providing a low cost sterile area for purposes of pharmaceutical or electronic production where a full clean room is not available, or needs to be supplemented.

In those prior sterile air trolleys air is drawn in to a base unit of the trolley by a fan and driven upwards through filters to a work surface of the trolley. In the case of US 4,531,956, the air exits through the work surface of the trolley, which is formed as a perforated upper face of a hollow platform, the platform also having perforations in its sides and in an overhanging underside of the platform. The filtered air thus passes out from the work surface in substantially all directions to create an envelope of sterile air surrounding the work surface. This envelope of filtered air will, however, be disrupted by the objects placed on the work surface and vulnerable to disturbance in the environment of the trolley, and in practice cannot be relied upon to maintain a sterile environment around the work surface in the run up to and during the course of an operation. A further risk of having airflow upwardly through outlets In the work surface is the entrainment of blood from surgical instruments replaced on the work surface in the airflow as an aerosol.

German Patent DE-2836708 discloses a mobile clean air generating trolley having an air outlet in its lower region, impeller means operative to move air into the inlet, through a filter and out of the trolley casing via an outlet unit located on top of the trolley work surface. The outlet unit is made of porous material and comprises a ring channel and a cushioned layer. The ring channel is hollow and extends fully around the perimeter of the work surface. Filtered air is directed into the outlet unit and is emitted upwardly through the porous material of the cushioned layer and outwardly from the porous walls of the ring channel, in all directions.

US 3 629 999 discloses a unit for providing an aseptic barrier over a target area, having a blower in a housing for drawing air through a filter, airflow being directed from the housing to an air plenum, an annular diffuser being centrally positioned within the plenum, a lip overhang being provided on the superior edge of the inner periphery of the diffuser. In use, air is directed through the diffuser, which discharges air in a 360° arc towards the centre of the diffuser well, the rushing masses of air colliding and forming a vertically rising vortex.

The subsequent US patent, US 4,996,910 seeks to improve on the earlier design and provides a horizontal work station on the top of the trolley having a casing upstanding from one end of the working surface with perforations to direct filtered air outwardly in all directions as well as having deflector plates to deflect bulk air flow from the upstanding casing substantially horizontally across the work surface toward the user. As with the earlier design, the objective is to create a buffer zone of sterile air extending substantially out beyond the main work surface and beyond the main sterile airflow in order to seek to prevent any inflow or entrainment of contaminants into the sterile zone. Whereas this approach is presented as an improvement on the earlier design, it cuts down on the available surface area of the work surface and access to the work surface in view of the upstanding casing and tall side panels, and there remain risks of entrainment of contaminants due to the horizontal flow of air

It is an objective of the present invention to provide an improved sterile air trolley that overcomes or mitigates problems of the prior sterile air trolleys, which is compact, efficient, effective and relatively economical to manufacture.

### Summary of the Invention

According to a first aspect of the present invention there is provided a sterile air trolley comprising a casing having at least one air inlet in its lower region and a plurality of air outlets in its upper region, and enclosing impeller means operative to move air in through the at least one inlet, through filter means and out of the casing by way of the outlets, the upper region of the casing providing a substantially horizontal work surface, the work surface having an upstanding boundary wall extending around its perimeter whereby the work surface and boundary wall form a tray, the boundary wall being hollow whereby the filtered air is directed into the boundary wall and is emitted through air outlets in the boundary wall, wherein there are suitably no air outlets directed upwardly or outwardly, characterised in that the boundary wall comprises four sides, the outlets facing inwardly of the boundary wall such that the filtered air is directed over the work surface in use from opposing sides, forming a continuously replenished rising layer of filtered air over the work surface, wherein the air flows from all four sides and rises as a column as the air flows merge and wherein the trolley casing is in a modular form having a base unit housing the impeller and an upper unit comprising the tray-shaped work surface and boundary wall, the upper unit being readily demountable from and re- mountable to the base unit.

The sterile/filtered air is directed across the work surface from all directions inwardly of the boundary wall and forms a continuously replenished rising layer of filtered air over the work surface within the sterile zone / volume defined by the boundary wall. The continuously replenished rising layer of filtered air prevents any inflow/entrainment of contaminants into the sterile zone.

Compared to the prior configurations of sterile air trolley, the working surface in the apparatus of the present invention is protected by a sterile air layer that is robust while not significantly constricting the available work surface area or impeding access to it.

The shape of the tray is suitably rectangular, with the boundary wall defining the four sides of the rectangle and wherein the filtered air is emitted inwardly over the work surface from all four sides.

The upper unit may suitably mount to the base unit in a push-fit or friction fit manner or have releasable fastening means. The compact nature of the upper unit enables it to be carried independently of the trolley base unit and placed in a sterilising facility such as an autoclave, sterilising by steam, radiant energy or other suitable means. Indeed, this opens up the possibility for the work surface of the trolley and the surgical instruments to be sterilised alongside each other to facilitate preparation of the equipment prior to a surgical operation.

In a particularly preferred embodiment of the invention, the sterilised surgical instruments are set out on the work surface of the upper unit and sealed in by a film, foil or lid of barrier material that is mounted above the work surface. A hinged or detachable lid is preferred but a barrier foil may, for example, be stretched over the top of the work surface at the level of the upper edge of the boundary wall and be adhered to the boundary wall by releasable means to enable access to the surgical instruments at the start of the operation.

Preferably the height of the boundary wall is slightly greater than the height of the any of the instruments or other items placed flat on the work surface of the tray in order to fully enshroud those instruments. Furthermore, for a typical surgical tray of approximately 500mm x 700mm in area, it is appropriate to have a minimum height of boundary wall of the order of 90mm. The height limits are suitably measured as the height of the wall that is provided with air outlets.

An optimal maximum height for this area of tray work surface is of the order of 200 to 300mm to maintain the blanket of sterile air without greatly increasing the capacity and power consumption of the impeller.

A preferred rate of flow of air from the outlets is of the order of 0.4 to 0.5 meters per second, and suitable no less than 0.35 meters per second. This lower limit of flow rate is appropriate to prevent the thermal environment surrounding the trolley, including any convection air- flow, from disrupting the air blanket formed over the work surface of the tray. The upper limit helps to restrict entrainment.

Particularly preferably, that part of the boundary wall comprising the air outlets is in the form of a mesh or is densely perforated with many substantially uniformly distributed apertures to provide a substantially uniform flow of air through the boundary wall.

The construction of the part of the boundary wall comprising the outlets is suitably such as to provide a pressure drop of the order of at least 10 pascals and suitably greater than 20 pascals and particularly preferably of the order of 30 - 50 pascals. In one preferred embodiment a mesh providing a 38 pascal drop is used.

According to a second aspect of the present invention there is provided a sterile air cabinet comprising a casing having at least one air inlet in its lower region and a plurality of air outlets in its upper region, and enclosing impeller means operative to move air in through the at least one inlet, through filter means and out of the casing by way of the outlets, the upper region of the casing providing a substantially horizontal work surface, characterised in that the work surface has an upstanding boundary wall extending around its perimeter whereby the work surface and boundary wall form a tray, the boundary wall being hollow whereby the filtered air is directed into the boundary wall and is emitted through air outlets in the boundary wall facing inwardly over the work surface from opposing sides.

According to a further aspect of the present invention there is provided a sterile air tray having a work surface with an upstanding boundary wall extending around its perimeter, the boundary wall being hollow whereby filtered air may be directed into the boundary wall and emitted through air outlets in the boundary wall facing inwardly over the work surface from opposing sides.

### Brief Description of the Drawings

A preferred embodiment of the present invention will now be more particularly described by way of example with reference to the accompanying drawings, wherein:

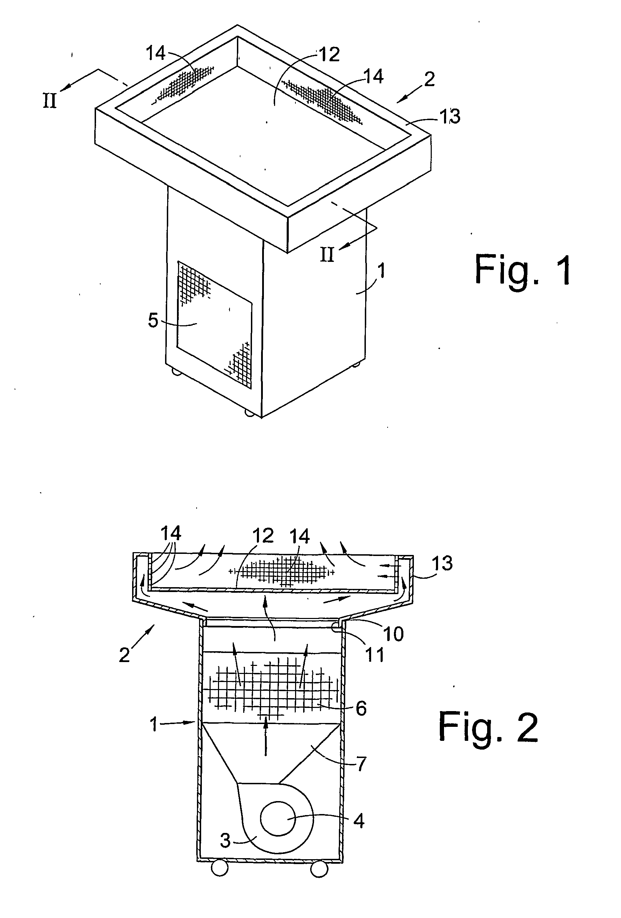

Figure 1 is a perspective view of a sterile air trolley embodying the invention; and
Figure 2 is a sectional view through the sterile air trolley of Figure 1 taken along a line II-II in Figure 1.

### Description of the Preferred Embodiment

The illustrated sterile air trolley comprises a mobile casing assembled in two modular parts comprising a base unit 1 and an upper unit 2.

The base unit 1 incorporates the fan 3 with its associated impeller 4 to draw air in through an inlet 5 in the casing of the base unit 1. This air is directed upwardly and distributed against the lower face of a filter unit 6 by a manifold 7. The filter unit 6 is a HEPA filter in the form of a rectangular block that spans the hollow interior of the cabinet of the base unit 1 and through which all of the fan driven air must pass, filtering out particulate contaminants in the air.

At the upper end of the casing of the base unit 1, the upper rim 10 of the casing serves as a seat for the upper module 2 of the trolley. A mating lower rim 11 of the upper unit 2 push fits into the upper rim 10 of the base unit 1, providing a fluid tight seal whereby filtered air flowing up through the base unit 1 is directed straight into the interior of the upper unit 2. Releasable clips or clamps (not shown) may be provided to secure the upper unit 2 in place on the base unit 1.

The lower part of the upper unit 2 is formed as a continuation of the cabinet of the base unit 1, suitably having substantially the same cross sectional area of hollow interior and being formed of a sheet metal such as stainless steel. Choice of stainless steel as preferred material for construction at least of the upper unit 2 is to suit the objective of being able to steam sterilise/autoclave the upper unit 2.

As viewed from above or to the side, the upper unit 2 takes the shape of a tray having a floor that serves as a work surface 12 on to which the surgical instruments or other items to be kept in a sterile environment may be placed.

The perimeter of the work surface 12 is bounded by an upstanding boundary wall 13. Since the work surface 12 is rectangular, the boundary wall 13 extends around the four sides of the rectangle.

The boundary wall 13 is hollow and receives air flowing up from the filter unit 6. The lower part of the upper unit 2 serves as a manifold/expansion section to duct the filtered air around the underside of the work surface 12 and up into the wall 13 from all sides. For the avoidance of doubt, although the boundary wall is described as a single continuous hollow wall it can, in principle, be internally partitioned as four separate opposing walls or in any other manner provided that the airflow is not too greatly disrupted and that there is a substantially uniform flow of air out through the wall/walls 13.

The filtered air flows out through a multitude of apertures 14 on the inner face of the wall(s) 13 directing the air inwardly generally toward the centre of the tray. The air flows in from all four sides and rises as a column as the air flows merge. A continuously replenished rising layer/column of filtered air is thus provided within the sterile air zone defined by the boundary wall 13 of the tray shaped upper unit 2.

The inner face of each opposing length of the wall 13 may be formed as a mesh panel and is suitably a stainless steel mesh/grid, the apertures of the grid lattice work each being a respective air outlet 14. The air outlet apertures 14 are substantially uniformly spaced and distributed throughout the extent of each of the opposing lengths of the inner face of the wall 13 to allow substantially uniform airflow over the work surface 12. Any reduction in air flow that would otherwise arise from, for example, the opposing end wall lengths of the boundary wall 13 being farther apart than the side wall lengths of the boundary wall 13, can be compensated for by varying the pressure drop across the wall 13 lengths. As a generality, the mesh is suitably adapted to provide a pressure drop of the order of 38 pascals but this may be adjusted along the lengths of the walls 13 if necessary. The rate of flow of air from the boundary wall 13 inwardly over the work surface 12 is suitably a substantially uniform 0.4m per second.

Although not shown in detail, the boundary wall 13 may have the apertured inner face/mesh separately formed from the stainless steel sheet that otherwise defines the boundary wall 13 and with a part of the stainless steel or an additional element forming a frame structure to support the mesh. Conversely, instead of having a grid/mesh, the inner face of the boundary wall 13 may be a continuation of the stainless steel sheet which is perforated in a dense and uniform manner to provide the multitude of air outlets.

In the illustrated preferred embodiment the tray form of the upper unit 2 has a rectangular floor area of 500 x 700mm and a boundary wall height of 90 mm. This corresponds to the typical work surface area of a surgical instrument tray/trolley. The surgeon or his assistants have ready access to any surgical implements laid on the work surface 12, the boundary wall 13 does not impede access and yet is of sufficient height to maintain a robust blanket of sterile air over all of the .surgical implements placed on the work surface 12.

Unlike the prior art, in the preferred embodiment there are suitably no air apertures directed upwardly or outwardly so as to provide sterile air in a broad range beyond the sterile zone. Without any such additional air outlets the arrangement of the present invention functions well and generally better than when any external air outlets are provided.

Although in the illustrated embodiment the tray part of the apparatus is shown as being a rigid construction of fixed wall 13 height, and with the apertures 14 distributed over the full height of the wall 13, in alternative embodiments the outlets 14 may be provided for only part of the height of the boundary wall 13 but suitably still to a height of 90 mm with the apertures being predominantly in the upper region of the wall. Accordingly, the full height of the boundary wall may be greater. Additionally, the boundary wall 13 could be adapted to have a telescopic construction to selectively increase or decrease the height of the boundary wall 13 so that the height of the wall 13 may be increased when to be used with relatively large bulky tools such as hip operation drills and the like.

The demountability of the upper unit 2 from the base unit 1 provides the user with a great deal of versatility in management of the apparatus. As noted previously, this enables the user, for example, to sterilise the work surface 12 along with the surgical instruments and enables the instruments to be placed ready for use on the work surface 12 and carried with the upper unit 2 back to the base unit 1 for reassembly of the upper unit 2 to the base unit 1.

Although not shown in the illustrated embodiment, the apparatus may be provided with additional air conditioning facilities such as, for example an air cooler or heater. Such facilities, if required, are suitably installed in the base unit 1. The base unit 1 may also be provided with a power transformer or, preferably, a battery for fully independent operation, making the apparatus fully self-contained and mobile.

## Claims

1. A sterile air trolley comprising a casing having at least one air inlet (5) in its lower region and a plurality of air outlets (14) in its upper region, and enclosing impeller means (4) operative to move air in through the at least one inlet (5), through filter means (6) and out of the casing by way of the outlets (14), the upper region of the casing providing a substantially horizontal work surface (12), the work surface (12) having an upstanding boundary wall (13) extending around its perimeter whereby the work surface and boundary wall form a tray, the boundary wall (13) being hollow whereby the filtered air is directed into the boundary wall (13) and is emitted through air outlets in the boundary wall, wherein there are suitably no air outlets directed upwardly or outwardly, **characterised in that** the boundary wall comprises four sides, the outlets facing inwardly of the boundary wall such that the filtered air is directed over the work surface in use from opposing sides, forming a continuously replenished rising layer of filtered air over the work surface, wherein the air flows from all four sides and rises as a column as the air flows merge and wherein the trolley casing is in a modular form having a base unit (1) housing the impeller (4) and an upper unit (2) comprising the tray-shaped work surface (12) and boundary wall (13), the upper unit being readily demountable from and re-mountable to the base unit.

2. A sterile air trolley as claimed in claim 1, wherein the sterile/filtered air is directed across the work surface from all directions inwardly of the boundary wall and forms a continuously replenished rising layer of filtered air over the work surface within the sterile zone / volume defined by the boundary wall.

3. A sterile air trolley as claimed in claim 1 or 2, wherein the shape of the tray is rectangular, with the boundary wall defining the four sides of the rectangle and wherein the filtered air is emitted inwardly over the work surface from all four sides.

4. A sterile air trolley as claimed in any preceding claim, wherein the sterilised surgical instruments are set out on the work surface of the upper unit (2) and sealed in by a film, foil or lid of barrier material that is mounted above the work surface (12).

5. A sterile air trolley as claimed in any preceding claim, wherein an optimal maximum height for this area of tray work surface is of the order of 200 to 300mm to maintain the blanket of sterile air without greatly increasing the capacity and power consumption of the impeller.

6. A sterile air trolley as claimed in claim 5, wherein a preferred rate of flow of air from the outlets is of the order of 0.4 to 0.5 meters per second, and suitably no less than 0.35 meters per second.

7. A sterile air trolley as claimed in claim 6, wherein that part of the boundary wall comprising the air outlets is in the form of a mesh or is densely perforated with many substantially uniformly distributed apertures to provide a substantially uniform flow of air through the boundary wall.

8. A sterile air trolley as claimed in claim 7, wherein the construction of the part of the boundary wall comprising the outlets is such as to provide a pressure drop of the order of at least 10 pascals.

9. A sterile air trolley according to any preceding claim wherein the casing is a mobile casing.

## Patentansprüche

1. Sterilluft-Wagen mit einem Gehäuse, dass zumindest einen Lufteinlass (5) in seinem unteren Bereich und eine Vielzahl von Luftauslässen (14) in seinem oberen Bereich umfasst und Gebläseeinrichtungen (4) umschließt, die betreibbar sind, um Luft durch den zumindest einen Einlass (5) durch Filtereinrichtungen (6) hindurch in das Gehäuse und aus diesem heraus über Auslässe (14) zu bewegen, wobei der obere Bereich des Gehäuses eine im Wesentlichen horizontale Arbeitsoberfläche (12) bildet, wobei die Arbeitsoberfläche (12) eine nach oben vorstehende Begrenzungswand (13) aufweist, die sich um den Umfang der Arbeitsoberfläche (12) erstreckt, wodurch die Arbeitsoberfläche und die Begrenzungswand eine Wanne bilden, wobei die Begrenzungswand (13) hohl ist, wodurch die gefilterte Luft in die Begrenzungswand (13) gelenkt wird und durch Luftauslässe in der Begrenzungswand ausgestoßen wird, worin in geeigneter Weise keine Luftauslässe nach oben oder nach außen gerichtet sind, **dadurch gekennzeichnet, dass** die Begrenzungswand vier Seiten umfasst, dass die Auslässe zur Innenseite der Begrenzungswand gerichtet sind, derart, dass die gefilterte Luft über die Arbeitsoberfläche im Gebrauch von entgegengesetzten Seiten gelenkt wird, wodurch eine kontinuierlich ergänzte ansteigende Schicht von gefilterter Luft über der Arbeitsoberfläche gebildet wird, wobei die Luft von allen vier Seiten strömt und als eine Säule ansteigt, während sich die Luftströmungen vereinigen, und wobei das Wagen-Gehäuse eine modulare Form aufweist, die eine Basiseinheit (1), in der das Gebläse untergebracht ist, und eine obere Einheit (2) aufweist, die die wannenförmige Arbeitsoberfläche (12) und die Begrenzungswand (13) umfasst, wobei die obere Einheit einfach von der Basiseinheit abbaubar und wieder an diese anbaubar ist.

2. Sterilluft-Wagen nach Anspruch 1, bei dem die sterile/gefilterte Luft von allen Richtungen einwärts von der Begrenzungswand über die Arbeitsoberfläche gerichtet wird und eine kontinuierlich ergänzte ansteigende Schicht von gefilterter Luft über der Arbeitsoberfläche innerhalb der sterilen Zone/des sterilen Volumens bildet, das durch die Begrenzungswand begrenzt ist.

3. Sterilluft-Wagen nach Anspruch 1 oder 2, bei dem die Form der Wanne rechtwinklig ist, wobei die Begrenzungswand vier Seiten des Rechteckes bildet, und bei dem die gefilterte Luft von allen vier Seiten nach innen über die Arbeitsoberfläche ausgestoßen wird.

4. Sterilluft-Wagen nach einem der vorhergehenden Ansprüche, bei dem die sterilisierten chirurgischen Instrumente auf der Arbeitsoberfläche der oberen Einheit (2) ausgelegt sind und durch einen über der Arbeitsoberfläche (12) befestigten Film, eine Folie oder einen Deckel aus Sperrmaterial versiegelt sind.

5. Sterilluft-Wagen nach einem der vorhergehenden Ansprüche, bei dem eine optimale maximale Höhe für diesen Bereich der Wannen-Arbeitsoberfläche in der Größenordnung von 200 bis 300mm ist, um Abdeckung aus steriler Luft aufrechtzuerhalten, ohne die Kapazität und den Leistungsverbrauch des Gebläses stark zu vergrößern.

6. Sterilluft-Wagen nach Anspruch 5, bei dem die bevorzugte Strömungsrate der Luft aus den Auslässen in der Größenordnung von 0,4 bis 0,5 Meter pro Sekunde und in geeigneter Weise nicht kleiner als 0,35 Meter pro Sekunde ist.

7. Sterilluft-Wagen nach Anspruch 6, bei dem der Teil der Begrenzungswand, der die Luftauslässe aufweist, die Form eines Siebes hat oder dicht perforiert mit vielen im Wesentlichen gleichförmig verteilten Öffnungen ist, um eine im Wesentlichen gleichförmige Luftströmung durch die Begrenzungswand zu schaffen.

8. Sterilluft-Wagen nach Anspruch 7, bei dem die Konstruktion des Teils der Begrenzungswand, der die Auslässe umfasst, derart ist, dass sich ein Druckabfall in der Größenordnung von zumindest 10 Pascal ergibt.

9. Sterilluft-Wagen nach einem der vorhergehenden Ansprüche, bei dem das Gehäuse ein mobiles Gehäuse ist.

## Revendications

1. Chariot à air stérile comprenant un boîtier ayant au moins une entrée d'air (5) dans sa région inférieure et une pluralité de sorties d'air (14) dans sa région supérieure, et entourant des moyens de turbine (4) opérationnels pour faire entrer l'air par la au moins une entrée (5) en passant par des moyens de filtre (6) et le faire sortir du boîtier au moyen des sorties (14), la région supérieure du boîtier fournissant une surface de travail (12) sensiblement horizontale, la surface de travail (12) ayant une paroi de limite droite (13) s'étendant autour de son périmètre, moyennant quoi la surface de travail et la paroi de limite forment un plateau, la paroi de limite (13) étant creuse, moyennant quoi l'air filtré est dirigé dans la paroi de limite (13) et est émis par les sorties d'air dans la paroi de limite, dans lequel il n'y a pas, de manière appropriée, de sorties d'air dirigées vers le haut ni vers l'extérieur, **caractérisé en ce que** la paroi de limite comprend quatre côtés, les sorties étant orientées vers l'intérieur de la paroi de limite de sorte que l'air filtré est dirigé sur la surface de travail, à l'usage, à partir des côtés opposés, formant une couche montante réapprovisionnée en continu d'air filtré sur la surface de travail, dans lequel l'air s'écoule à partir des quatre côtés et remonte comme une colonne lorsque les écoulements d'air fusionnent et dans lequel le boîtier de chariot se présente sous une forme modulaire ayant une unité de base (1) logeant la turbine (4) et une unité supérieure (2) comprenant la surface de travail en forme de plateau (12) et la paroi de limite (13), l'unité supérieure étant facilement démontable de et remontable sur l'unité de base.

2. Chariot à air stérile selon la revendication 1, dans lequel l'air stérile/filtré est dirigé sur la surface de travail à partir de toutes les directions vers l'intérieur de la paroi de limite et forme une couche montante d'air filtré réapprovisionnée en continu sur la surface de travail à l'intérieur de la zone/volume stérile défini(e) par la paroi de limite.

3. Chariot à air stérile selon la revendication 1 ou 2, dans lequel la forme du plateau est rectangulaire, avec la paroi de limite qui définit les quatre côtés du rectangle et dans lequel l'air filtré est émis vers l'intérieur sur la surface de travail à partir des quatre côtés.

4. Chariot à air stérile selon l'une quelconque des revendications précédentes, dans lequel les instruments chirurgicaux stérilisés sont placés sur la surface de travail de l'unité supérieure (2) et rendus étanches par un film, une feuille ou un couvercle de matériau formant barrière qui est monté au-dessus de la surface de travail (12).

5. Chariot à air stérile selon l'une quelconque des revendications précédentes, dans lequel une hauteur maximum optimale pour cette zone de surface de travail de plateau est de l'ordre de 200 à 300 mm afin de maintenir la couverture d'air stérile sans augmenter considérablement la capacité ni la consommation de courant de la turbine.

6. Chariot à air stérile selon la revendication 5, dans lequel le débit d'air préféré des sorties est de l'ordre de 0,4 à 0,5 mètre par seconde, et de manière appropriée, non inférieur à 0,35 mètre par seconde.

7. Chariot à air stérile selon la revendication 6, dans lequel cette partie de la paroi de limite comprenant les sorties d'air se présente sous la forme d'un filet ou est perforée de manière dense avec de nombreuses ouvertures réparties de manière sensiblement uniforme afin de fournir un écoulement d'air sensiblement uniforme à travers la paroi de limite.

8. Chariot à air stérile selon la revendication 7, dans lequel la construction de la partie de la paroi de limite comprenant les sorties est telle qu'elle fournit une chute de pression de l'ordre d'au moins 10 Pascals.

9. Chariot à air stérile selon l'une quelconque des revendications précédentes, dans lequel le boîtier est un boîtier mobile.
